# FASCICULE DE BREVET EUROPEEN

(11) **EP 0 635 495 B1**
(45) Date de publication et mention de la délivrance du brevet: **16.09.1998**
(21) Numéro de dépôt: 94401547.8
(22) Date de dépôt: 06.07.1994
(51) Int. Cl.: C07D 235/02, C07D 263/52, A61K 31/415, A61K 31/42

(54) **Dérivés de benzospiroalcène, leur procédé de préparation et les compositions pharmaceutiques qui les contiennent**
Benzospiroalken-Derivate, Verfahren zu ihrer Herstellung und diese enthaltende pharmazeutische Zusammensetzungen
Benzospiroalkene derivates, process for their preparation and pharmaceutical compositions containing them

(30) Priorité: 20.07.1993 FR 9308859
(43) Date de publication de la demande: 25.01.1995
(73) Titulaire: ADIR ET COMPAGNIE, 92415 Courbevoie Cédex (FR)
(72) Inventeur: Cordi, Alex, F-92150 Suresnes (FR); Lacoste, Jean-Michel, F-92310 Sevres (FR); Laubie, Michel, F-92420 Vaucresson (FR); Verbeuren, Tony, F-78540 Vernouillet (FR); Descombes, Jean-Jacques, F-93360 Neuilly-Plaisance (FR); Millan, Mark, F-75017 Paris (FR)

(56) Documents cités:
- JOURNAL OF MEDICINAL CHEMISTRY., vol.21, no.6, 1978, WASHINGTON US pages 585 - 587 P. A. CROOKS ET AL 'Synthesis of spiro(tetralin-2,2'-pyrrolidine) and spiro(indan-2,2'-pyrrolidine) derivatives as potential analgesics'
- JOURNAL OF THE CHEMICAL SOCIETY, PERKIN TRANSACTIONS 1., no.11, 1979, LETCHWORTH GB pages 2719 - 2726 P. A. CROOKS ET AL 'Synthesis of 5-hydroxy and 5,6-dihydroxy-derivatives of spiro(indane-2,2'-pyrrolidine), rigid analogues of tyramine and dopamine respectively'

## Description

La présente invention concerne de nouveaux benzospiroalcènes, leur procédé de préparation et les compositions pharmaceutiques qui les contiennent, ainsi que leur utilisation en tant qu'agonistes α₂ adrénergiques.

Le système nerveux adrénergique joue un rôle important à plusieurs niveaux, par exemple artériel, veineux, cardiaque, rénal et au niveau du système nerveux autonome central et périphérique. Dès lors, les produits capables d'intéragir avec les récepteurs adrénergiques peuvent induire un grand nombre de réponses physiologiques comme la vasoconstriction, la vasodilatation, l'augmentation ou la diminution du rythme cardiaque, la variation de la force de contraction du muscle cardiaque et des activités métaboliques. Différents composés adrénergiques ont été utilisés dans le passé pour modifier ces réponses physiologiques ou d'autres.

La stimulation adrénergique dans le système nerveux périphérique est thérapeutiquement utile lorsqu'une constriction vasculaire a lieu telle que dans les congestions nasales, optiques ou ophtalmiques et dans l'inflammation. Dans le système nerveux central, la stimulation adrénergique est particulièrement utile pour induire l'analgésie, l'anesthésie et la diurèse ainsi que pour traiter l'hypertension et les symptômes de sevrage aux opiacés. Ces effets sont notamment décrits par P. TIMMERMANS et coll. dans "Comprehensive Medicinal Chemistry" (Vol. III, p. 134-185, 1990 - C. HANSH editor, Pergamon, Oxford, 1990).
Les composés les plus proches de l'Art antérieur sont plus particulièrement les composés décrits dans J. Med. Chem., 1978, 21, 6, 585-587 et dans J. Chem Soc., Perkin Tr-1, 1979, 11, 2719-2726.

Les composés décrits dans la présente invention, outre le fait qu'ils soient nouveaux, possèdent un profil d'agonistes α₂ adrénergiques qui les rend utiles, comme indiqué, entre autres, par P. TIMMERMANS et coll. (J. Med. Chem., 25, n° 12, 1389-1401, 1982) ou S. MUNK et coll. (Bioorg. & Med. Chem Lett., 4, n° 3, 459-462, 1994), comme inhibiteurs de la suractivation des voies centrales supposées contribuer à l'anxiété et aux attaques de panique, en tant qu'anesthésiques (afin de réduire les besoins en agents d'inhalation et de promouvoir la stabilité hémodynamique sans dépression respiratoire), en tant qu'analgésiques (plus particulièrement dans le traitement des douleurs neuropathiques), ainsi qu'en tant qu'hypotenseurs, sédatifs, vasoconstricteurs, décongestionnants, hypotenseurs oculaires et pour remédier aux symptômes de l'abstinence opiacée. L'utilité thérapeutique des produits de l'invention se base sur leur sélectivité pour les récepteurs adrénergiques et leur modulation sélective des fonctions adrénergiques dans différents tissus et organes.
Plus spécifiquement la présente invention concerne les composés de formule (I) : 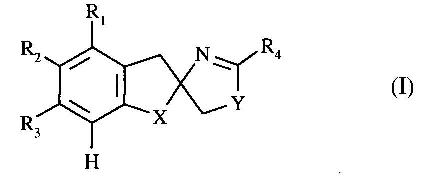 dans laquelle :
- - X: représente -CH₂-, -(CH₂)₂-, -CH=CH-, -O-CH₂-, -S-CH₂-, -SO-CH₂- ou -SO₂-CH₂-,
- - Y: représente un atome d'oxygène, de soufre, ou un groupement -NR₅-,
- - R₁: représente un atome d'halogène, un groupement alkyle (C₁-C₆) linéaire ou ramifié (substitué ou non par un ou plusieurs atomes d'halogène), un groupement hydroxy ou un groupement alkoxy (C₁-C₆)linéaire ou ramifié,
- - R₂: représente un atome d'hydrogène, d'halogène, un groupement alkyle (C₁-C₆) linéaire ou ramifié (substitué ou non par un ou plusieurs atomes d'halogène), un groupement hydroxy, un groupement alkoxy (C₁-C₆) linéaire ou ramifié ou un groupement alkylthio (C₁-C₆) linéaire ou ramifié,
- - R₃: représente un atome d'hydrogène, d'halogène, un groupement alkyle (C₁-C₆) linéaire ou ramifié (substitué ou non par un ou plusieurs atomes d'halogène), un groupement hydroxy, un groupement alkoxy (C₁-C₆) linéaire ou ramifié ou un groupement alkylthio (C₁-C₆) linéaire ou ramifié,
- - R₄: représente un atome d'hydrogène ou un groupement amino (substitué ou non par un ou deux groupements alkyles (C₁-C₆) linéaire ou ramifié),
- - R₅: représente un atome d'hydrogène ou un groupement alkyle (C₁-C₆) linéaire ou ramifié,
ou bien
- R₁ et R₂ forment ensemble avec les atomes de carbone qui les portent un cycle benzénique à la condition que dans ce cas X représente -CH₂- ou
   -(CH₂)₂-,
leurs isomères ainsi que leurs sels d'addition à un acide pharmaceutiquement acceptable.

Parmi les acides pharmaceutiquement acceptables on peut citer à titre non limitatif les acides chlorhydrique, bromhydrique, sulfurique, phosphorique, acétique, lactique, malonique, succinique, fumarique, tartrique, maléïque, citrique, méthane sulfonique, etc...

Parmi les isomères éventuels des composés de formule (I), on peut citer les énantiomères, les diastéréoisomères, les épimères ainsi que les tautomères.

L'invention s'étend également au procédé de préparation des composés de formule (I).

Les composés de formule (I) pour lesquels Y représente un groupement -NR₅ sont obtenus selon le procédé caractérisé en ce que l'on utilise comme produit de départ un composé de formule (II) : 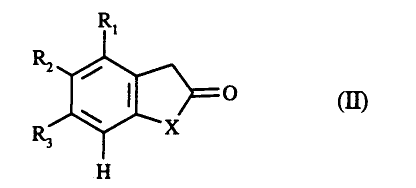 dans laquelle X, R₁, R₂ et R₃ ont la même signification que dans la formule (I)
que l'on fait réagir :
- soit avec de la benzylamine en présence d'acide paratoluènesulfonique pour conduire au composé de formule (III) : 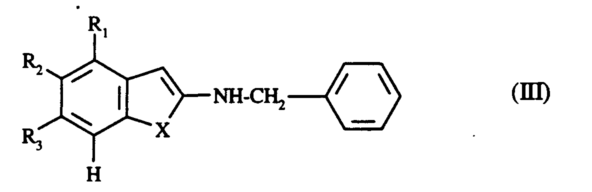 dans laquelle X, R₁, R₂ et R₃ ont la même signification que dans la formule (I)
   que l'on fait réagir, sous atmosphère inerte, avec du cyanure de triméthylsilyle en présence d'iodure de zinc,
   pour conduire au composé de formule (IV) : 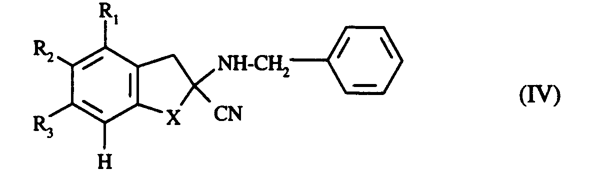 dans laquelle X, R₁, R₂ et R₃ ont la même signification que dans la formule (I),
   que l'on réduit à l'aide d'hydrure de lithium aluminium, puis par hydrogénation catalytique pour conduire au composé de formule (V) : 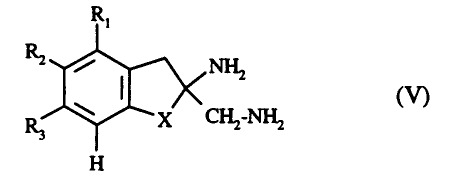 dans laquelle X, R₁, R₂ et R₃ ont la même signification que dans la formule (I),
- soit avec du cyanure de potassium en présence de chlorure d'ammonium en milieu inerte ou avec du cyanure de sodium en milieu acide, ou bien avec du cyanure de triméthylsilyle en présence d'iodure de zinc puis avec une solution alcoolique saturée en ammoniac,
   pour conduire au composé de formule (VI) : 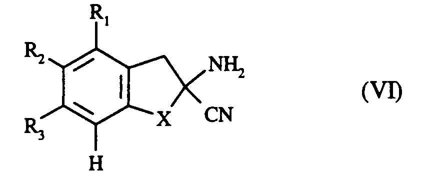 dans laquelle X, R₁, R₂ et R₃ ont la même signification que dans la formule (I),
   que l'on réduit à l'aide d'hydrure de lithium aluminium pour conduire au composé de formule (V) décrit précédemment,
   composé de formule (V),
   que l'on fait réagir avec de la formamidine en milieu alcoolique, un formiate d'alkyle ou avec un halogènure de cyanogène (suivie, selon la nature du composé de formule (I) que l'on souhaite obtenir, d'une réaction d'alkylation à l'aide d'un halogénure d'alkyle),
   pour conduire au composé de formule (I/a), cas particulier des composés de formule (I) : 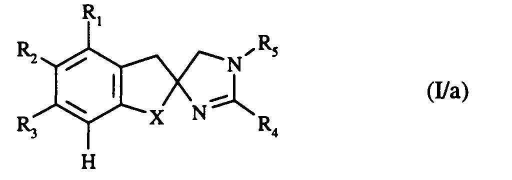 dans laquelle X, R₁, R₂, R₃, R₄ et R₅ ont la même signification que dans la formule (I)
   composé de formule (I/a),
   que l'on purifie, le cas échéant, selon une technique classique de purification,
   dont on sépare, si on le souhaite, les isomères selon une technique classique de purification, et que l'on transforme, éventuellement, en leurs sels d'addition à un acide pharmaceutiquement acceptable.

Les composés de formule (I) pour lesquels Y représente un atome d'oxygène ou de soufre sont obtenus selon le procédé caractérisé en ce que l'on utilise comme produit de départ un composé de formule (VI) décrit précédemment,
que l'on fait réagir avec de l'acide formique en milieu anhydre saturé en acide chlorhydrique, pour conduire au composé de formule (VII) : 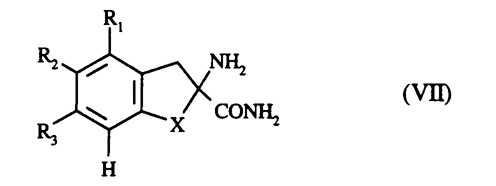 dans laquelle R₁, R₂, R₃ et X ont la même signification que dans la formule (I),
que l'on transforme en acide correspondant de formule (VIII) en milieu chlorhydrique concentré : 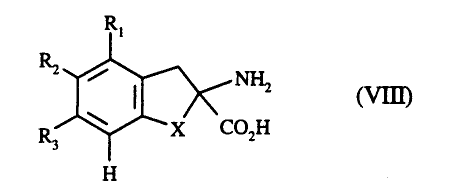 dans laquelle R₁, R₂, R₃ et X ont la même signification que dans la formule (I),
qui subit une réduction par l'hydrure de lithium aluminium en milieu inerte,
pour conduire au composé de formule (IXa) : 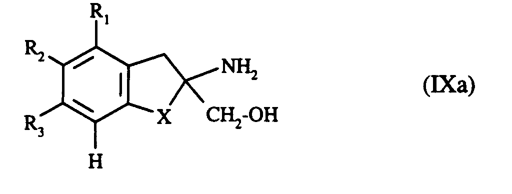 dans laquelle R₁, R₂, R₃ et X ont la même signification que dans la formule (I),
composé de formule (IXa), que l'on transforme, selon la nature des composés de formule (I) que l'on souhaite obtenir, en tosylate correspondant à l'aide d'acide p.toluènesulfonique puis que l'on fait réagir avec de la thiourée ou de l'acide thioacétique,
pour conduire, après hydrolyse, au composé de formule (IXb) : 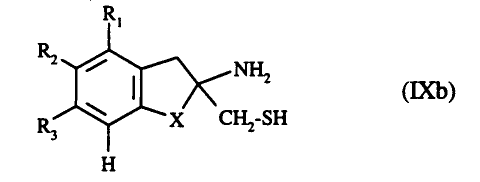 dans laquelle R₁, R₂, R₃ et X ont la même signification que dans la formule (I),
composé de formule (IXa) ou (IXb) que l'on fait réagir avec de la formamidine en milieu alcoolique, un formiate d'alkyle ou avec un halogénure de cyanogène (suivie, selon la nature du composé de formule (I) que l'on souhaite obtenir, d'une réaction d'alkylation à l'aide d'un halogénure d'alkyle),
pour conduire au composé de formule (I/b), cas particulier des composés de formule (I) : 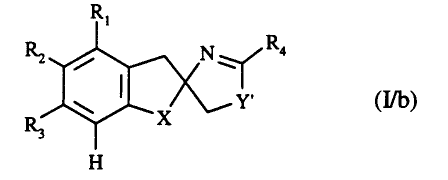 dans laquelle R₁, R₂, R₃, R₄ et X ont la même signification que précédemment et Y' représente un atome d'oxygène ou de soufre,
composé de formule (I/b)
que l'on purifie, le cas échéant, selon une technique classique de purification,
dont on sépare, si on le souhaite, les isomères selon une technique classique de purification,
et que l'on transforme, éventuellement, en leurs sels d'addition à un acide pharmaceutiquement acceptable.

Lorsque les composés de formule (I) que l'on souhaite obtenir possèdent en R₁, R₂ ou R₃ un groupement hydroxy, un procédé préférentiel d'obtention de ces composés consiste à synthétiser dans un premier temps le dérivé de formule (I) possédant en R₁, R₂ ou R₃ un groupement alkoxy que l'on transforme en groupement hydroxy correspondant par action du tribromure de bore en milieu dichlorométhane.

La présente invention a également pour objet les compositions pharmaceutiques renfermant comme principe actif au moins un composé de formule générale (I) ou un de ses sels d'addition à un acide pharmacologiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiées, les comprimés sublingaux, les gélules, les tablettes, les suppositoires, les crèmes, pommades, gels dermiques, etc...

La posologie varie selon l'âge et le poids du patient, la nature et la sévérité de l'affection ainsi que la voie d'administration.

Celle-ci peut être orale, nasale, rectale ou parentérale. D'une manière générale, la posologie unitaire s'échelonne entre 0,1 et 1000 mg pour un traitement en 1 à 3 prises par 24 heures.

Les exemples suivants illustrent l'invention. Les produits de départ utilisés sont des produits connus ou préparés selon des modes opératoires connus.

### Exemple 1: Spiro[(1,3-diazacyclopent-1-ène)-5: 2'-(8'-chloro-1',2',3',4'-tétrahydronaphtalène)], fumarate

### Stade A: 2-Amino-2-cyano-8-chloro-1,2,3,4-tétrahydronaphtalène

A une solution fortement agitée et maintenue sous azote contenant 43 mmoles de 8-chloro-3,4-dihydronaphtalèn-2(1H)-one dans 60 ml d'éthanol et 30 ml d'eau sont ajoutées successivement 44 mmoles de cyanure de potassium et 44 mmoles de chlorure d'ammonium. Après 20 heures d'agitation à 20°C, le mélange est concentré sous vide et le résidu repris par 80 ml d'acétate d'éthyle. Cette phase organique est lavée par de l'eau puis extraite par de l'acide chlorhydrique 1N. La phase aqueuse est alcalinisée par de la soude à 35 % et extraite par de l'acétate d'éthyle. Le produit attendu est obtenu après séchage et évaporation de la phase organique sous forme solide.
Point de fusion : 67-69°C

### Stade B: 2-Amino-2-aminométhyl-8-chloro-1,2,3,4-tétrahydronaphtalène

A une suspension contenant 43 mmoles d'hydrure de lithium aluminium dans 50 ml de tétrahydrofurane est additionnée, goutte à goutte, une solution contenant 19 mmoles du composé obtenu au stade précédent, en maintenant une température ne dépassant pas 20°C. Le mélange est agité 30 minutes, refroidi à 0°C puis hydrolysé par addition de 1,6 ml d'eau, 1,6 ml de soude 2N puis 3,5 ml d'eau. La suspension résultante est filtrée et le filtrat évaporé pour conduire au produit attendu sous forme d'huile.

### Stade C: Spiro[(1,3-diazacyclopent-1-ène)-5 : 2'-(8'-chloro-1',2',3',4'-tétrahydronaphtalène)], fumarate

Un mélange contenant 14 mmoles du composé obtenu au stade précédent et 14 ml d'acétate de formamidine dans 60 ml d'éthanol est agité, à 20°C, sous atmosphère d'azote, pendant 10 heures. Le solvant est alors évaporé et le résidu repris par de l'acide chlorhydrique 1N. Cette phase acide est lavée par de l'acétate d'éthyle et alcalinisée par de la soude à 35 %. Le mélange est alors extrait par de l'acétate d'éthyle, la phase organique est lavée par une solution saturée de chlorure de sodium et évaporée. Le résidu solide ainsi obtenu est dissous dans 20 ml d'éthanol traité par un équivalent d'acide fumarique dissous dans de l'éthanol. Après évaporation du solvant, le produit attendu est obtenu par recristallisation du résidu dans de l'éthanol.
Point de fusion : 213-215°C

| Microanalyse élémentaire : | | | | |
|---|---|---|---|---|
| | C % | H % | N % | Cl % |
| calculé | 57,06 | 5,09 | 8,32 | 10,53 |
| trouvé | 56,88 | 5,21 | 8,30 | 10,47 |

Les exemples 2, 3 et 4 ont été synthétisés selon le procédé décrit dans l'exemple 1 en utilisant les produits de départ correspondants.

### Exemple 2: Spiro[(1,3-diazacyclopent-1-ène)-5 : 2'-(8'-fluoro-1',2',3',4'-tétrahydronaphtalène)], fumarate

Point de fusion : 187-190°C

| Microanalyse élémentaire : | | | |
|---|---|---|---|
| | C % | H % | N % |
| calculé | 60,00 | 5,35 | 8,75 |
| trouvé | 59,89 | 5,60 | 8,46 |

### Exemple 3: Spiro[(1,3-diazacyclopent-1-ène)-5 : 2'-(8'-méthoxy-1',2',3',4'-tétrahydronaphtalène)], fumarate

Point de fusion : 186-188°C

| Microanalyse élémentaire : | | | |
|---|---|---|---|
| | C % | H % | N % |
| calculé | 61,44 | 6,07 | 8,43 |
| trouvé | 61,30 | 5,90 | 8,55 |

### Exemple 4: Spiro[(1,3-diazacyclopent-1-ène)-5 : 2'-(6',8'-dichloro-1',2',3',4'-tétrahydronaphtalène)], fumarate

Point de fusion : 194-195°C

| Microanalyse élémentaire : | | | | |
|---|---|---|---|---|
| | C % | H % | N % | Cl % |
| calculé | 51,77 | 4,34 | 7,55 | 19,10 |
| trouvé | 51,80 | 4,37 | 7,43 | 18,54 |

### Exemple 5: Spiro[(1,3-diazacyclopent-1-ène)-5 : 2'-(8'-hydroxy-1',2',3',4'-tétrahydronaphtalène)], fumarate

A une solution maintenue sous azote contenant 4,5 mmoles du composé décrit dans l'exemple 3 dans 30 ml de dichlorométhane, sont additionnées, goutte à goutte, 16,6 ml d'une solution 1M de tribromure de bore dans le dichlorométhane. La température du milieu réactionnel est amenée à 20°C puis le mélange est versé sur une solution glacée de bicarbonate de sodium. Après évaporation de la phase aqueuse, le résidu est repris par de l'isopropanol. Le solvant est évaporé et l'huile obtenue est purifiée par chromatographie sur colonne de silice en utilisant comme éluant un mélange eau/dioxane/ammoniaque (90/10/1). Le solide isolé est dissous dans de l'éthanol et traité par un équivalent d'acide fumarique en solution dans de l'éthanol. Le produit attendu est obtenu après évaporation et recristallisation du résidu dans de l'éthanol.
Point de fusion : 240-242°C

| Microanalyse élémentaire : | | | |
|---|---|---|---|
| | C % | H % | N % |
| calculé | 64,60 | 6,20 | 10,76 |
| trouvé | 63,95 | 6,11 | 10,58 |

### Exemple 6: Spiro[(1,3-diazacyclopent-1-ène)-5 : 2'-(8'-chloro-1',2',3',4'-tétrahydronaphtalène)], L(+)tartrate, isomère α

Le composé de l'exemple 1 est dédoublé au moyen d'acide L(+)tartrique par recristallisations successives dans le méthanol. La pureté énantiomérique est vérifiée par chromatographie sur colonne chirale α₁-AGP en utilisant comme éluant un mélange Na₂HPO₄aq.0,01M/NaH₂PO₄aq.0,01M/n-propanol (60/40/1).
Point de fusion : 218-221°C

| Microanalyse élémentaire : | | | | |
|---|---|---|---|---|
| | C % | H % | N % | Cl % |
| calculé | 51,83 | 5,16 | 7,56 | 9,56 |
| trouvé | 51,27 | 5,02 | 7,54 | 9,56 |

### Exemple 7: Spiro[(1,3-diazacyclopent-1-ène)-5 : 2'-(8'-chloro-1',2',3',4'-tétrahydronaphtalène)], D(-)tartrate, isomère β

Le produit attendu est obtenu selon le procédé décrit dans l'exemple 6 à partir du composé de l'exemple 1 et d'acide D(-)tartrique.
Point de fusion : 218-221°C

| Microanalyse élémentaire : | | | | |
|---|---|---|---|---|
| | C % | H % | N % | Cl % |
| calculé | 51,83 | 5,16 | 7,56 | 9,56 |
| trouvé | 51,88 | 5,16 | 7,64 | 9,64 |

### Exemple 8: Spiro[(1,3-diazacyclopent-1-ène)-5 : 3'-(1',2',3',4'-tétrahydrophénanthrène)], fumarate

### Stade A: 3-Amino-3-cyano-1,2,3,4-tétrahydrophénanthrène

A un mélange, sous agitation, contenant 13 mmoles de 1,2-dihydro-phénanthrèn-3(4H)-one et 40 mmoles de cyanure de sodium dans 70 ml d'eau et 10 ml d'éther éthylique, est ajouté, goutte à goutte, 1 ml d'acide chlorhydrique concentré. Après 1 heure d'agitation à 20°C, la phase organique est décantée, lavée à l'eau, séchée et concentrée sous vide. L'huile résiduelle est traitée par 20 ml d'une solution méthanolique d'ammoniac (3,5 M), sous agitation, en milieu fermé, pendant 4 heures, à 20°C. Après évaporation du solvant, l'huile obtenue est reprise par 30 ml d'éther éthylique, et extraite par de l'acide chlorhydrique 1N. La phase aqueuse est alcalinisée par de la soude à 35 % puis extraite par de l'éther éthylique. Après séchage et évaporation, le produit attendu est obtenu sous forme solide.
Point de fusion : 75-78°C

### Stade B: 3-Amino-3-aminométhyl-1,2,3,4-tétrahydrophénanthrène

Le produit attendu est obtenu selon le procédé décrit au stade B de l'exemple 1, à partir du composé décrit au stade précédent.

### Stade C: Spiro[(1,3-diazacyclopent-1-ène)-5 : 3'-(1',2',3',4'-tétrahydrophénanthrène)], fumarate

Le produit attendu est obtenu selon le procédé décrit au stade C de l'exemple 1, à partir du composé décrit au stade précédent.
Point de fusion : 212-215°C

| Microanalyse élémentaire : | | | |
|---|---|---|---|
| | C % | H % | N % |
| calculé | 68,17 | 5,72 | 7,95 |
| trouvé | 67,78 | 5,63 | 7,93 |

### Exemple 9: Spiro[(1-oxa-2-amino-3-azacyclopent-2-ène)-4 : 2'-(8'-chloro-1',2',3',4'-tétrahydronaphtalène)], chlorhydrate

### Stade A: 2-Amino-2-cyano-8-chloro-1,2,3,4-tétrahydronaphtalène

A une solution fortement agitée et maintenue sous azote contenant 277 mmoles de 8-chloro-3,4-dihydro-naphtalèn-2(1H)-one dans 350 ml de méthanol et 170 ml d'eau, sont ajoutées successivement 282 mmoles de cyanure de potassium et 290 mmoles de chlorure d'ammonium. Après 48 heures d'agitation à 20°C, le mélange est concentré. Le résidu est repris par de l'acétate d'éthyle. Cette phase organique est lavée à l'eau, extraite par de l'acide chlorhydrique 1N. Les phases acides sont alcalinisées par de la soude à 35 % et extraites par de l'acétate d'éthyle. Le produit attendu est obtenu après séchage et évaporation des phases organiques, sous forme solide.
Point de fusion : 67-69°C

### Stade B: 2-Amino-2-aminocarbonyl-8-chloro-1,2,3,4-tétrahydronaphtalène, chlorhydrate

Une solution contenant 114 mmoles du composé obtenu au stade précédent dans 110 ml d'acide formique est refroidie à 0°C et saturée par de l'acide chlorhydrique gazeux et anhydre pendant 3 heures. Le mélange est alors agité, à 20°C, pendant 16 heures, le solvant est évaporé et le résidu repris par 150 ml d'acétone. Le produit attendu est obtenu par filtration du solide blanc cristallisé.

### Stace C: Acide 2-amino-8-chloro-1,2,3,4-tétrahydronaphtalène-2-carboxylique

Une suspension contenant 79 mmoles du composé obtenu au stade précédent dans 125 ml d'acide chlorhydrique 6N est portée à reflux jusqu'à dissolution complète. Le solvant est alors évaporé, le résidu repris par de l'isopropanol et le pH de la solution amené à 7 par addition de soude 1N. Le produit attendu est obtenu après filtration et séchage du solide blanc précipité.

### Stade D: 2-Amino-2-hydroxyméthyl-8-chloro-1,2,3,4-tétrahydronaphtalène

A une solution contenant 35,4 mmoles du composé obtenu au stade précédent dans 100 ml de tétrahydrofurane (THF) est ajoutée, goutte à goutte, sous atmosphère d'azote, à température ambiante, une suspension de 79 mmoles d'hydrure de lithium aluminium dans 150 ml de THF anhydre. Le mélange est porté 1 heure au reflux. Après refroidissement à 0°C, le milieu est hydrolysé par addition successive de 3 ml d'eau, 3 ml de soude 2,5 N et 6 ml d'eau. Le solide blanc formé est filtré et lavé par du THF. Le produit attendu est obtenu, sous forme d'huile, après concentration des filtrats sous vide.

### Stade E: Spiro[(1-oxa-2-amino-3-azacyclopent-2-ène)-4 : 2'-(8'-chloro-1',2',3',4'-tétrahydronaphtalène)], chlorhydrate

Une solution contenant 33,5 mmoles de bromure de cyanogène dans 15 ml de dichlorométhane est additionnée, à 0°C, à une solution contenant 29,5 mmoles du composé obtenu au stade précédent dans 60 ml de dichlorométhane. L'ensemble est agité 16 heures à 20°C puis le solide formé est filtré et lavé par du dichlorométhane. Les filtrats sont lavés par une solution de bicarbonate de potassium, séchés et évaporés. Le produit attendu est obtenu par purification du résidu par chromatographie sur colonne de silice en utilisant comme éluant un mélange dichlorométhane/éthanol/ ammoniaque (92,5/7/0,5). L'huile obtenue est reprise par de l'éther éthylique et traitée par une solution d'éther chlorhydrique (4N). Le précipité formé est filtré et recristallisé dans un mélange isopropanol/éther éthylique.
Point de fusion : 210-214°C

| Microanalyse élémentaire : | | | | |
|---|---|---|---|---|
| | C % | H % | N % | Cl % |
| calculé | 52,76 | 5,17 | 10,26 | 25,96 |
| trouvé | 52,99 | 5,10 | 10,16 | 25,71 |

### Exemple 10 : Spiro[(1-oxa-2-amino-3-azacyclopent-2-ène)-4 : 2'-(7',8'-dichloro-1',2',3',4'-tétrahydronaphtalène)], chlorhydrate

Le produit attendu est obtenu selon le procédé décrit dans l'exemple 9 en utilisant le produit de départ correspondant.
Point de fusion : > 260°C

| Microanalyse élémentaire : | | | | |
|---|---|---|---|---|
| | C % | H % | N % | Cl % |
| calculé | 46,86 | 4,26 | 9,11 | 34,58 |
| trouvé | 46,63 | 4,45 | 8,89 | 34,97 |

### Exemple 11: Spiro[(1-oxa-2-amino-3-aacyclopent-2-ène)-4 : 2'-(7',8'-diméthyl-1',2',3',4'-tétrahydronaphtalène)], chlorhydrate

Le produit attendu est obtenu selon le procédé décrit dans l'exemple 9 en utilisant le produit de départ correspondant.
Point de fusion : 246-249°C

| Microanalyse élémentaire : | | | | |
|---|---|---|---|---|
| | C % | H % | N % | Cl % |
| calculé | 63,03 | 7,18 | 10,50 | 13,29 |
| trouvé | 62,00 | 7,03 | 10,31 | 13,08 |

### Exemple 12: Spiro[(1-oxa-2-amino-3-azacyclopent-2-ène)-4 : 2'-(8'-chloro-1',2',3',4'-tétrahydronaphtalène)], chlorhydrate, isomère α

### Stade A : 2-Amino-8-hydroxyméthyl-8-chloro-1,2,3,4-tétrahydronaphtalène, fumarate, isomère α

Le produit attendu est obtenu par dédoublement du composé obtenu au stade D de l'exemple 9 au moyen d'acide (+)-dibenzoyl-D-tartrique par recristallisations successives dans l'éthanol. La pureté énantiomérique est contrôlée par chromatographie chirale sur colonne DIACEL-AD en utilisant comme éluant un mélange isopropanol/n-heptane/diéthylamine (40/1000/0,8). Le sel est alors partagé entre la soude 9N et le dichlorométhane. La phase aqueuse est extraite par du dichlorométhane. Après séchage et évaporation des phases organiques, le résidu est dissous dans de l'éthanol, avec un équivalent d'acide fumarique et l'ensemble est porté à reflux. Le produit attendu est alors obtenu après refroidissement sous forme d'un solide blanc qui est filtré.

### Stade B : Spiro[(1-oxa-2-amino-3-azacyclopent-2-ène)-4 : 2'-(8'-chloro-1',2',3',4'-tétrahydronaphtalène)], chlorhydrate, isomère α

Le produit attendu est obtenu selon le procédé décrit au stade E de l'exemple 9 à partir du composé obtenu au stade précédent.
Point de fusion : 212-214°C

| Microanalyse élémentaire : | | | | |
|---|---|---|---|---|
| | C % | H % | N % | Cl % |
| calculé | 52,76 | 5,17 | 10,26 | 25,96 |
| trouvé | 52,09 | 5,18 | 10,01 | 25,88 |

### Exemple 13: Spiro[(1-oxa-2-amino-3-azacyclopent-2-ène)-4 : 2'-(8'-chloro-1',2',3',4'-tétrahydronaphtalène)], chlorhydrate, isomère β

### Stade A : 2-Amino-8-hydroxyméthyl-8-chloro-1,2,3,4-tétrahydronaphtalène, fumarate, isomère β

Le produit attendu est obtenu selon le procédé décrit au stade A de l'exemple 12 en utilisant pour le dédoublement l'acide (-)dibenzoyl-L-tartrique.

### Stade B : Spiro[(1-oxa-2-amino-3-azacyclopent-2-ène)-4 : 2'-(8'-chloro-1',2',3',4'-tétrahydronaphtalène)], chlorhydrate, isomère β

Le produit attendu est obtenu selon le procédé décrit au stade E de l'exemple 9 à partir du composé obtenu au stade précédent.
Point de fusion : 212-214°C

| Microanalyse élémentaire : | | | | |
|---|---|---|---|---|
| | C % | H % | N % | Cl % |
| calculé | 52,76 | 5,17 | 10,26 | 25,96 |
| trouvé | 52,51 | 5,20 | 10,03 | 25,62 |

### Etude pharmacologique des composés de l'invention

### Exemple 14: Etude in vitro sur les artères fémorales et les veines saphènes de chien

La technique utilisée s'inspire de celle décrite par FOWLER et Coll. (J. Pharmacol. Exp. Ther., 229, 712-718, 1984). Des chiens bâtards mâles ou femelles d'environ 15-25 kg ont été utilisés comme source d'organes. Les animaux sont anesthésiés au pentobarbital (30 mg/kg, en intraveineux). Les pattes sont incisées et les vaisseaux prélevés. Ils sont placés dans du liquide de Krebs-Ringer (NaCl 118 mM ; NaHCO₃ 25 mM ; Glucose 10 mM ; KCl 4,7 mM; CaCl₂ 1,25 mM ; MgSO₄ 1,19 mM ; KH₂PO₄ 1,14 mM) à température ambiante et sous bullage de carbogène (95 % 0₂, 5 % CO₂). Ces vaisseaux sont alors soigneusement débarrassés de leur graisse puis découpés en anneaux de 2 mm de large et montés sous une tension de base de 4 g (artères fémorales) ou 1 g (veines saphènes) dans des cuves thermostatées à 37°C contenant du liquide de Krebs-Ringer constamment bullé par du carbogène. Un crochet inférieur constitue le point fixe, tandis que le crochet supérieur est relié à un capteur de force isométrique. Les variations de tension sont digitalisées, stockées sur disque et traitées par un système informatique. Après montage, les organes sont laissés en repos pendant 90 minutes, des rinçages étant effectués toutes les 30 min. Après réajustement de la tension de base, une contraction est provoquée par une dose unique de KCl (100 mM). Après stabilisation, lavage et retour à la ligne de base, une contraction est provoquée par une dose unique de phényléphrine (concentration submaximale) afin de régulariser les contractions suivantes. Après lavage et retour à la ligne de base, une courbe effet/concentration est réalisée par une adjonction de doses cumulatives d'agoniste (l'espacement entre les doses est d'un demi-log). Cette expérience permet de calculer la concentration efficace 50 % (EC₅₀) de la manière suivante : les valeurs de tension sont d'abord converties en pourcentages par rapport à l'effet maximum provoqué par 100 mM de KCl. Cette EC₅₀ est déterminée par régression non linéaire par la méthode SIMPLEX (M.S. CACECI, Byte, 340-362, 1984), calculée suivant le modèle de la loi d'action de masse de L. MICHAELIS et M.L. MENTEN (Biochem. Zeitschrifft, 49, 333-369, 1913).
E = (Emax*Cⁿ)(ECⁿ+Cⁿ) avec E = effet ; Emax = effet maximum;
C = concentration ; EC = EC₅₀ ; n = nombre de HILL

Les produits de l'invention contractent les artères et les veines de chien. Le maximum de ces contractions se rapproche de celui obtenu avec le KCl. Les résultats obtenus sont présentés dans le tableau ci-dessous :

| Exemple | ARTERE | | VEINE | |
|---|---|---|---|---|
| | EC₅₀ (µM) | Max (% KCl) | EC₅₀ (µM) | Max (% KCl) |
| 1 | 1 | 94 | 0,2 | 95 |
| 3 | 3 | 94 | 0,4 | 100 |
| 6 | 2 | 91 | 7 | 96 |

### Exemple 15 : Etude in vivo chez le RAT AMYELE

Des rats Sprague Dawley mâles (300-400 g) sont anesthésiés à l'éther. La trachée est canulée, la moelle épinière est détruite au moyen d'une tige en acier et l'animal est immédiatement mis sous respiration artificielle. Les nerfs vagues sont sectionnés. Les artères carotides sont ligaturées, un cathéter est placé dans l'une et sert à enregistrer la pression artérielle. Trois autres cathéters sont placés dans les veines jugulaires et la veine du pénis et servent aux injections. La température des animaux est maintenue à 36°C. L'animal est prétraité par une injection de tertatolol (100 µg/kg). L'animal est également prétraité 10 minutes après par le prazosin (100 µg/kg) ou la yohimbine (1 mg/kg) lorsque l'on veut déterminer les propriétés alpha₁ ou alpha₂-adrénergiques du produit. Dix minutes plus tard, des doses cumulatives croissantes de produit sont injectées toutes les 20 secondes. Les variations de pression artérielles sont détectées à l'aide d'une cellule de pression P23XL Statham et sont enregistrées. Les valeurs de pression sont exprimées en mm Hg. Cette expérience permet de calculer la concentration augmentant la pression de 20 mm Hg (C₂₀) par régression non linéaire suivant le modèle de la loi d'action de masse de Michaelis et Menten comme décrit ci-dessus. L'effet maximum obtenu est ensuite converti en pourcentage par rapport à l'effet maximum provoqué par la phényléphrine. Les composantes alpha₁ ou alpha₂-adrénergiques du produit sont apppréciées à l'aide du rapport des C₂₀ obtenues en présence de prazosin ou de yohimbine sur les valeurs obtenues en absence de ces antagonistes. Chez le rat amyélé, les produits de l'invention produisent des hypertensions sensibles au prazosin et à la yohimbine. Les résultats sont rassemblés dans le tableau ci-dessous :

| Exemple | C₂₀ (µg/kg) | Ratio C₂₀ traité/C₂₀ contrôle | |
|---|---|---|---|
| | Contrôle | Prazosin | Yohimbine |
| 1 | 0,2 | 1,9 | 6,0 |
| 5 | 4,8 | 0,9 | 12,0 |
| 6 | 0,1 | 1,7 | 4,0 |

### Exemple 16: Effet de ligands α₂ adrénergiques sur l'activité de synthèse de la noradrénaline dans l'hippocampe de rat

Les rats mâles wistar (Iffa Credo, Illskischen, France) de 200-220 g sont stabulés individuellement dans des cages avec libre accès à la nourriture et l'eau de boisson. La température du laboratoire est de 21 ± 1°C et le pourcentage d'humidité de 60 ± 5 %. Le cycle jour/nuit est de 12 heures (lumière allumée le matin à 7 h 30).

### Analyse neurochimique

L'effet des molécules sur la vitesse de renouvellement de la noradrénaline est déterminé 60 minutes après leur administration par voie sous cutanée. L'activité des composés de l'invention a été comparée à celle d'un agoniste α₂ de référence, le UK 14,304 (Life Sciences, Vol. 43, n° 22, p.1805-1812, 1988). Trente minutes avant le sacrifice, les animaux reçoivent un inhibiteur de l'enzyme de décarboxylation, le NSD 1015 (100 mg/kg s.c.). Les animaux sont alors décapités, leur cerveau prélevé et l'hippocampe disséqué. L'hippocampe est ensuite homogénéisé dans 500 µl de HClO₄ 0,1M contenant 0,5 % de Na₂S₂O₅ et 0,5 % de EDTA disodique et centrifugés à 15000 g pendant 15 minutes à 4°C. Afin de mesurer les quantités de L-dihydroxyphénylalanine (L-DOPA), précurseur de la noradrénaline, présentes, les surnageants sont dilués 20 fois dans la phase mobile de chromatographie et 100 µl sont analysés par chromatographie liquide à haute performance et détection électrochimique (détecteur Waters M460, potentiel de l'électrode de travail, 850 mV). La colonne (Hypersil ODS 5 µm, C18, 150 x 4,6 mm, Spectra Physics) est thermostatée à 25°C. La phase mobile est composée de 100 mM de KH₂PO₄, 0,1 mM de EDTA,Na₂, 0,5 mM d'octylsulphonate de sodium et de 5 % de méthanol et ajustée à pH 3,15 avec du PO₄H₃ concentré. Le débit est de 1 ml/min. Les quantités de L-DOPA sont déterminées par rapport à une standardisation externe et rapportées à la quantité de protéine présente dans les hippocampes. Les protéines sont quantifiées selon la méthode de Smith et coll. (Anal. Biochem., 150, 76-85, 1985) avec comme standard la serum albumine bovine (Sigma Chemical Co., St Louis, MO). La quantité moyenne de L-DOPA analysée chez les animaux traités par le solvant est considérée comme valeur témoin (100 %). Les quantités analysées chez les animaux traités par les molécules sont alors exprimées en pourcentage par rapport à cette valeur témoin.

### Statistiques

Les données obtenues pour les animaux témoins et les animaux traités sont comparées par une analyse de variance (ANOVA) suivie d'un test de Dunnett. La limite de significativité est fixée à p < 0,05. Les données sont exprimées en moyenne ± écart type.

### Composés

Les injections sont faites par voie sous cutanée dans un volume de 1,0 ml.kg. Les substances sont dissoutes dans de l'eau stérile avec, si nécessaire, quelques gouttes d'acide lactique puis réajustés à un pH le plus proche possible de la neutralité avec de la soude. Le NSD-1015 est dissout dans du sérum physiologique. Les doses sont exprimées par rapport à leur poids de base libre.

Les résultats obtenus pour le composé de l'exemple 9 et le UK 14,304 sont présentés ci-dessous :

| | Dose (mg/kg) | Accumulation de L-DOPA Pourcentage des témoins |
|---|---|---|
| Exemple 9 | 0,01 | 51 ± 7* (n = 4) |
| | 0,04 | 50 ± 7* (n = 4) |
| | 0,16 | 44 ± 2* (n = 4) |
| | 0,63 | 34 ± 2* (n = 4) |
| UK 14,304 | 0,01 | 98 ± 13* (n = 4) |
| | 0,04 | 70 ± 2* (n = 4) |
| | 0,16 | 40 ± 3* (n = 4) |
| | 0,63 | 48 ± 7* (n = 4) |
| | Véhicule | 100 ± 5 (n = 4) |

| | | |
|---|---|---|
| * p < 0.05 par rapport au véhicule valeur absolue du L-DOPA : 476,4 ± 16,8 pg/mg protéine | | |

### Exemple 17 : Test de perte du réflexe de retournement chez le rat

### Matériels et méthodes

### Animaux et environnement

Les animaux utilisés sont des rats Wistar mâles (IFFA CREDO). Ils pèsent entre 200 et 250 g au moment de l'expérimentation. Les rats résident dans une animalerie, en cage, à quatre, avec libre accès à la nourriture et à la boisson, une semaine avant d'entrer en étude. 24 heures avant l'expérimentation, les rats sont mis à jeûn. Les rats sont transférés dans le laboratoire quelques heures avant l'expérimentation. L'animalerie et le laboratoire sont climatisés à une température de 21 ± 1°C et une hygrométrie de 55 ± 5 %. Le cycle d'éclairement est de 12 h/12 h avec la lumière allumée de 7 h jusqu'à 19 h. Les rats ne sont utilisés qu'une fois en expérimentation.

### Descriptif du test

Les composés sont injectés par voie sous-cutanée aux rats qui sont mis en cages individuelles. 30 minutes après l'injection, l'effet de ces produits sur le réflexe de retournement est observé et un score est attribué. Immédiatement après, les rats reçoivent une injection intrapéritonéale de xylazine à une dose de 40 mg/kg. 30 minutes après l'injection de xylazine ( = 60 minutes après l'injection des composés à tester), un score est de nouveau attribué au réflexe de retournement. Pour évaluer le réflexe de retournement, les rats sont placés doucement sur le dos puis lâchés. Un score de 0 est attribué à un retournement immédiat et complet, un score de 1 a un retournement sans reprise d'une position normale, un score de 2 a un essai de retournement mais le rat reste allongé sur le dos et un score de 3 a une perte complète du réflexe de redressement.

### Critères de jugement

Pour évaluer l'effet des composés testés sur la perte du réflexe de retournement, un score de 1 ou plus est considéré comme un effet agoniste. Par dose, le pourcentage d'animaux qui a un score de 1 ou plus est calculé, ainsi que la DE₅₀ (= dose à laquelle 50 % des animaux montrent un effet agoniste).

Pour évaluer l'effet des composés testés sur la perte du réflexe de retournement induite par la xylazine, un score de 2 ou moins est considéré comme un effet antagoniste. Par dose, le pourcentage d'animaux qui a un socre de 2 ou moins, est calculé ainsi qu'une DE₅₀ (= dose à laquelle 50 % des animaux montrent un effet antagoniste). Les DE₅₀ sont calculées selon Litchfield et Wilcoxon.

### Produits à tester

Les produits sont solubilisés dans l'eau distillée ou l'eau distillée avec quelques gouttes d'acide lactique en cas de difficulté de solubilisation. Le pH des solutions est alors amené à 5 à l'aide d'une solution de soude. Tous les produits sont administrés dans un volume de 10 ml/kg.

### Résultats

Chez les rats témoins, 30 minutes après l'injection souscutanée de l'eau distillée, aucune perte du réflexe de retournement n'était observée : score moyen ± SEM était de 0 ± 0 (N=10). 30 minutes après injection de la xylazine (et 60 minutes après injection de l'eau distillée en prétraitement), tous les rats témoins (N=10) avaient perdus le réflexe de retournement : score moyen ± SEM était de 3 ± 0. Le tableau ci-dessous montre les DE₅₀ des produits testés pour leur capacité, respectivement, induire une perte du réflexe de retournement ou à antagoniser une perte du réflexe de retournement induite par la xylazine. La xylazine, l'agoniste α₂ de référence UK 14,304 ainsi que les composés des exemples 9, 11 et 13 se comportent comme des agonistes en induisant une perte du réflexe de redressement à la différence de l'idazoxan, un antagoniste α₂ qui est inactif.

| Composés | Induction de la perte du réflexe de retournement | Antagonisme de la perte du réflexe de retournement induite par la xylazine |
|---|---|---|
| | DE₅₀ | DE₅₀ |
| XYLAZINE | 6,1 | - |
| UK 14,304 | 0,24 | > 10 |
| IDAZOXAN | > 10,0 | 0,24 |
| Exemple 9 | 0,17 | > 0,63 |
| Exemple 11 | 1,14 | > 2,50 |
| Exemple 13 | 0,15 | > 0,63 |

### Exemple 18: Composition pharmaceutique

| Formule de préparation pour 1000 comprimés dosés à 10 mg | |
|---|---|
| Composé de l'exemple 9 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 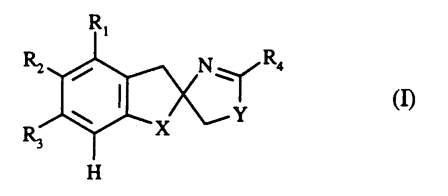 dans laquelle :
- X représente -CH₂-, -(CH₂)₂-, -CH=CH-, -O-CH₂-, -S-CH₂-, -SO-CH₂- ou -SO₂-CH₂-,
- Y représente un atome d'oxygène, de soufre, ou un groupement -NR₅-,
- R₁ représente un atome d'halogène, un groupement alkyle (C₁-C₆) linéaire ou ramifié (substitué ou non par un ou plusieurs atomes d'halogène), un groupement hydroxy ou un groupement alkoxy (C₁-C₆)linéaire ou ramifié,
- R₂ représente un atome d'hydrogène, d'halogène, un groupement alkyle (C₁-C₆) linéaire ou ramifié (substitué ou non par un ou plusieurs atomes d'halogène), un groupement hydroxy, un groupement alkoxy (C₁-C₆) linéaire ou ramifié ou un groupement alkylthio (C₁-C₆) linéaire ou ramifié,
- R₃ représente un atome d'hydrogène, d'halogène, un groupement alkyle (C₁-C₆) linéaire ou ramifié (substitué ou non par un ou plusieurs atomes d'halogène), un groupement hydroxy, un groupement alkoxy (C₁-C₆) linéaire ou ramifié ou un groupement alkylthio (C₁-C₆) linéaire ou ramifié,
- R₄ représente un atome d'hydrogène ou un groupement amino (substitué ou non par un ou deux groupements alkyles (C₁-C₆) linéaire ou ramifié),
- R₅ représente un atome d'hydrogène ou un groupement alkyle (C₁-C₆) linéaire ou ramifié,
ou bien
- R₁ et R₂ forment ensemble avec les atomes de carbone qui les portent un cycle benzénique à la condition que dans ce cas X représente -CH₂- ou
-(CH₂)₂-,
leurs isomères ainsi que leurs sels d'addition à un acide pharmaceutiquement acceptable.

2. Composés de formule (I) selon la revendication 1 tels que X représente -(CH₂)₂-, leurs isomères ainsi que leurs sels d'addition à un acide pharmaceùtiquement acceptable.

3. Composés de formule (I) selon la revendication 1 tels que Y représente -NH-, leurs isomères ainsi que leurs sels d'addition à un acide pharmaceutiquement acceptable.

4. Composés de formule (I) selon la revendication 1 tels que Y représente un atome d'oxygène, leurs isomères ainsi que leurs sels d'addition à un acide pharmaceutiquement acceptable.

5. Composés de formule (I) selon la revendication 1 tels que R₂ et R₃ représentent simultanément un atome d'hydrogène, leurs isomères ainsi que leurs sels d'addition à un acide pharmaceutiquement acceptable.

6. Composés de formule (I) selon la revendication 1 tels que R₄ représente un atome d'hydrogène, leurs isomères ainsi que leurs sels d'addition à un acide pharmaceutiquement acceptable.

7. Composé de formule (I) selon la revendication 1 tels que R₄ représente un groupement amino, leurs isomères ainsi que leurs sels d'addition à un acide pharmaceutiquement acceptable.

8. Composé de formule (I) selon la revendication 1 qui est le spiro[(1,3-diazacyclopent-1-ène)-5 : 2'-(8'-chloro-1',2',3',4'-tétrahydronaphtalène)], ses isomères ainsi que ses sels d'addition à un acide pharmaceutiquement acceptable.

9. Composé de formule (I) selon la revendication 1 qui est le spiro [(1-oxa-2-amino-3-azacyclopent-2-ène)-4 : 2'-(8'-chloro-1', 2',3',4'-tétrahydronaphtalène)], ses isomères ainsi que ses sels d'addition à un acide pharmaceutiquement acceptable.

10. Procédé de préparation des composés de formule (I) selon la revendication 1 pour lesquels Y représente un groupement -NR₅-, caractérisé en ce que l'on utilise comme produit de départ un composé de formule (II) : 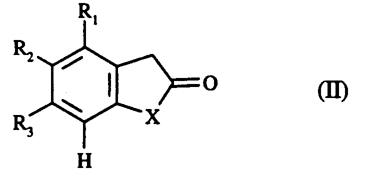 dans laquelle X, R₁, R₂ et R₃ ont la même signification que dans la formule (I)
que l'on fait réagir :
- soit avec de la benzylamine en présence d'acide paratoluènesulfonique pour conduire au composé de formule (III) : 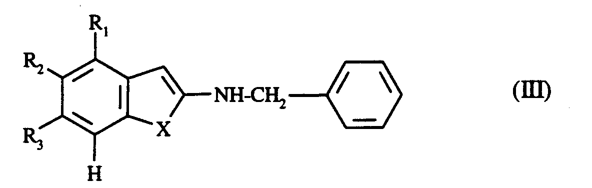 dans laquelle X, R₁, R₂ et R₃ ont la même signification que dans la formule (I)
que l'on fait réagir, sous atmosphère inerte, avec du cyanure de triméthylsilyle en présence d'iodure de zinc,
pour conduire au composé de formule (IV) : 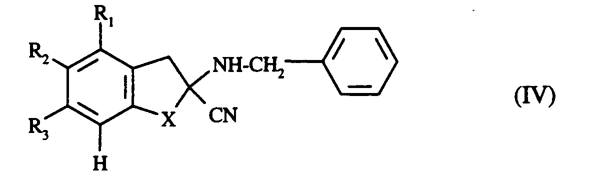 dans laquelle X, R₁, R₂ et R₃ ont la même signification que dans la formule (I),
que l'on réduit à l'aide d'hydrure de lithium aluminium, puis par hydrogénation catalytique pour conduire au composé de formule (V) : 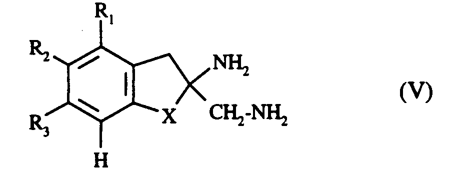 dans laquelle X, R₁, R₂ et R₃ ont la même signification que dans la formule (I),
- soit avec du cyanure de potassium en présence de chlorure d'ammonium en milieu inerte ou avec du cyanure de sodium en milieu acide, ou bien avec du cyanure de triméthylsilyle en présence d'iodure de zinc puis avec une solution alcoolique saturée en ammoniac,
pour conduire au composé de formule (VI) : 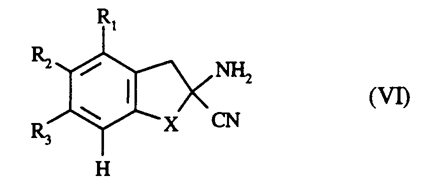 dans laquelle X, R₁, R₂ et R₃ ont la même signification que dans la formule (I),
que l'on réduit à l'aide d'hydrure de lithium aluminium pour conduire au composé de formule (V) décrit précédemment,
composé de formule (V),
que l'on fait réagir avec de la formanidine en milieu alcoolique, un formiate d'alkyle ou avec un halogènure de cyanogène (suivie, selon la nature du composé de formule (I) que l'on souhaite obtenir, d'une réaction d'alkylation à l'aide d'un halogénure d'alkyle),
pour conduire au composé de formule (I/a), cas particulier des composés de formule (I): 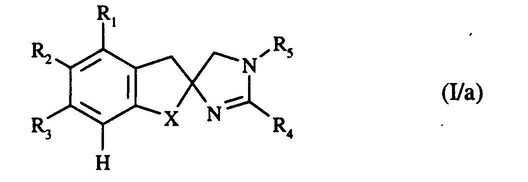 dans laquelle X, R₁, R₂, R₃, R₄ et R₅ ont la même signification que dans la formule (I)
composé de formule (I/a),
que l'on purifie, le cas échéant, selon une technique classique de purification,
dont on sépare, si on le souhaite, les isomères selon une technique classique de purification, et que l'on transforme, éventuellement, en leurs sels d'addition à un acide pharmaceutiquement acceptable.

11. Procédé de préparation des composés de formule (I) selon la revendication 1 pour lesquels Y représente un atome d'oxygène ou de soufre caractérisé en ce que l'on utilise comme produit de départ un composé de formule (VI) selon la revendication 10,
que l'on fait réagir avec de l'acide formique en milieu anhydre saturé en acide chlorhydrique, pour conduire au composé de formule (VII) : 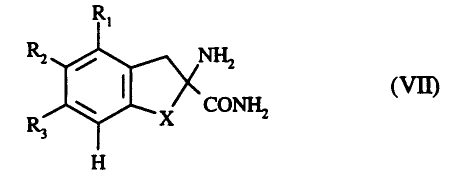 dans laquelle R₁, R₂, R₃ et X ont la même signification que dans la formule (I),
que l'on transforme en acide correspondant de formule (VIII) en milieu chlorhydrique concentré : 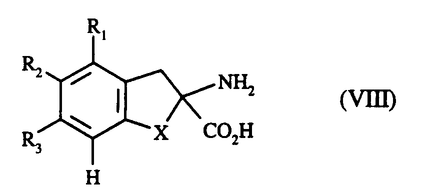 dans laquelle R₁, R₂, R₃ et X ont la même signification que dans la formule (I),
qui subit une réduction par l'hydrure de lithium aluminium en milieu inerte,
pour conduire au composé de formule (IXa) : 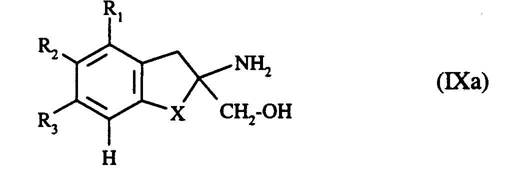 dans laquelle R₁, R₂, R₃ et X ont la même signification que dans la formule (I),
composé de formule (IXa), que l'on transforme, selon la nature des composés de formule (I) que l'on souhaite obtenir, en tosylate correspondant à l'aide d'acide p.toluènesulfonique puis que l'on fait réagir avec de la thiourée ou de l'acide thioacétique,
pour conduire, après hydrolyse, au composé de formule (IXb) : 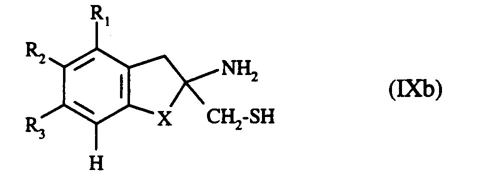 dans laquelle R₁, R₂, R₃ et X ont la même signification que dans la formule (I),
composé de formule (IXa) ou (IXb) que l'on fait réagir avec de la formamidine en milieu alcoolique, un formiate d'alkyle ou avec un halogénure de cyanogène (suivie, selon la nature du composé de formule (I) que l'on souhaite obtenir, d'une réaction d'alkylation à l'aide d'un halogénure d'alkyle),
pour conduire au composé de formule (I/b), cas particulier des composés de formule (I) : 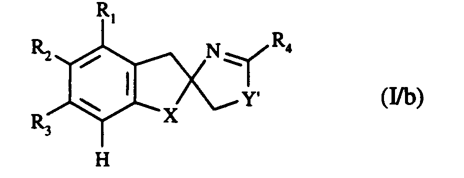 dans laquelle R₁, R₂, R₃, R₄ et X ont la même signification que précédemment et Y' représente un atome d'oxygène ou de soufre,
composé de formule (I/b)
que l'on purifie, le cas échéant, selon une technique classique de purification,
dont on sépare, si on le souhaite, les isomères selon une technique classique de purification,
et que l'on transforme, éventuellement, en leurs sels d'addition à un acide pharmaceutiquement acceptable.

12. Compositions pharmaceutiques contenant comme principe actif au moins un composé selon l'une quelconque des revendications 1 à 9 seul ou en combinaison avec un ou plusieurs excipients ou véhicules inertes, non toxiques, pharmaceutiquement acceptables.

13. Compositions pharmaceutiques selon la revendication 12 contenant au moins un principe actif selon l'une quelconque des revendications 1 à 9 utilisé comme agoniste α₂ adrénergique, en tant qu'anesthésique, analgésique, hypotenseur, sédatif, vasoconstricteur, décongestionnant et pour remédier aux symptômes de l'abstinence opiacée.

## Claims

1. Compounds of formula (I) : 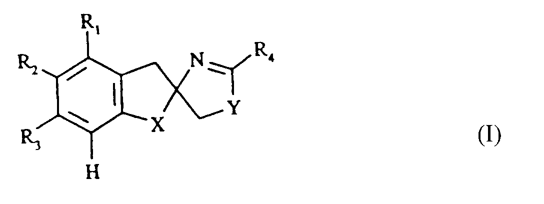 wherein :
- X represents -CH₂-, -( CH₂)₂-, -CH=CH-, -O-CH₂-, -S-CH₂-, -SO-CH₂- or -SO₂-CH₂-,
- Y represents an oxygen atom, a sulphur atom or an -NR₅- group,
- R₁ represents a halogen atom, a linear or branched (C₁-C₆)-alkyl group (unsubstituted or substituted by one or more halogen atoms), a hydroxy group or a linear or branched (C₁-C₆)-alkoxy group,
- R₂ represents a hydrogen atom, a halogen atom, a linear or branched (C₁-C₆)-alkyl group (unsubstituted or substituted by one or more halogen atoms), a hydroxy group, a linear or branched (C₁-C₆)-alkoxy group or a linear or branched (C₁-C₆)-alkylthio group,
- R₃ represents a hydrogen atom, a halogen atom, a linear or branched (C₁-C₆)-alkyl group (unsubstituted or substituted by one or more halogen atoms), a hydroxy group, a linear or branched (C₁-C₆)-alkoxy group or a linear or branched (C₁-C₆)-alkylthio group,
- R₄ represents a hydrogen atom or an amino group (unsubstituted or substituted by one or two linear or branched (C₁-C₆)-alkyl groups),
- R₅ represents a hydrogen atom or a linear or branched (C₁-C₆)-alkyl group,
or
- R₁ and R₂, together with the carbon atoms carrying them, form a benzene ring, with the proviso that in that case X represents -CH₂- or -(CH₂)₂-,
their isomers and also the addition salts thereof with a pharmaceutically acceptable acid.

2. Compounds of formula (I) according to claim 1 wherein X represents -(CH₂)₂-, their isomers, and also the addition salts thereof with a pharmaceutically acceptable acid.

3. Compounds of formula (I) according to claim 1 wherein Y represents -NH-, their isomers, and also the addition salts thereof with a pharmaceutically acceptable acid.

4. Compounds of formula (I) according to claim 1 wherein Y represents an oxygen atom, their isomers, and also the addition salts thereof with a pharmaceutically acceptable acid.

5. Compounds of formula (I) according to claim 1 wherein R₂ and R₃ simultaneously represent a hydrogen atom, their isomers, and also the addition salts thereof with a pharmaceutically acceptable acid.

6. Compounds of formula (I) according to claim 1 wherein R₄ represents a hydrogen atom, their isomers, and also the addition salts thereof with a pharmaceutically acceptable acid.

7. Compounds of formula (I) according to claim 1 wherein R₄ represents an amino group, their isomers, and also addition salts thereof with a pharmaceutically acceptable acid.

8. Compound of formula (I) according to claim 1 which is spiro[5-(1,3-diazacyclopent-1-ene) : 2'-(8'-chloro-1',2',3',4'-tetrahydronaphthalene)], its isomers, and also the addition salts thereof with a pharmaceutically acceptable acid.

9. Compound of formula (I) according to claim 1 which is spiro[4-(1-oxa-2-amino-3-azacyclopent-2-ene) : 2'-(8'-chloro-1',2',3',4'-tetrahydronaphthalene)], its isomers, and also the addition salts thereof with a pharmaceutically acceptable acid.

10. Process for the preparation of compounds of formula (I) according to claim 1 wherein Y represents an -NR₅- group, characterised in that there is used as starting material a compound of formula (II) : 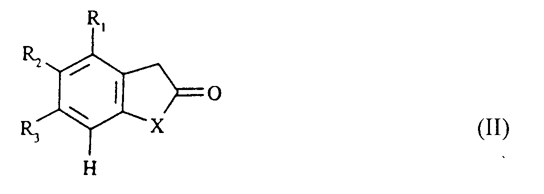 wherein X, R₁, R₂ and R₃ are as defined for formula (I),
which is reacted :
- either with benzylamine in the presence of para-toluenesulphonic acid to yield a compound of formula (III) : 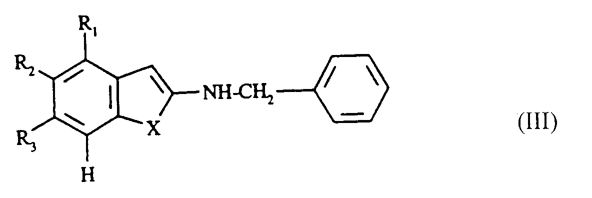 wherein X, R₁, R₂ and R₃ are as defined for formula (I),
which is reacted in an inert atmosphere with trimethylsilyl cyanide in the presence of zinc iodide
to yield a compound of formula (IV) : 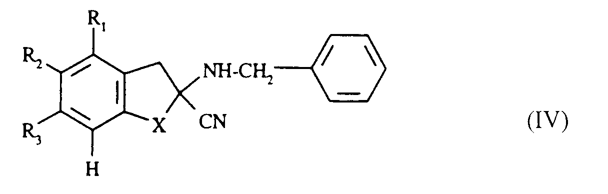 wherein X, R₁, R₂ and R₃ are as defined for formula (I),
which is reduced with lithium aluminium hydride and then by catalytic hydrogenation to yield a compound of formula (V) 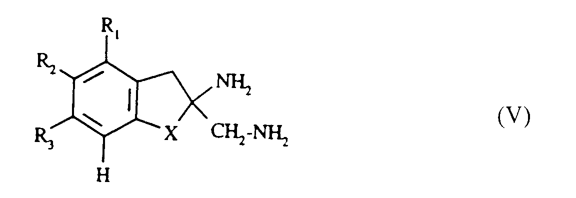 wherein X, R₁, R₂ and R₃ are as defined for formula (I),
- or with potassium cyanide in the presence of ammonium chloride in an inert medium, or with sodium cyanide in acid medium, or with trimethylsilyl cyanide in the presence of zinc iodide then with a saturated solution of ammonia in alcohol,
to yield a compound of formula (VI) : 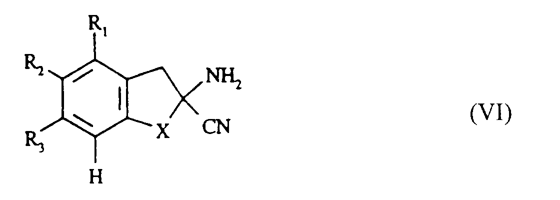 wherein X, R₁, R₂ and R₃ are as defined for formula (I),
which is reduced with lithium aluminium hydride to yield a compound (V) described above,
which compound of formula (V)
is reacted with formamidine in alcoholic medium, an alkyl formate or with a cyanogen halide (followed, depending on the nature of the desired compound of formula (I), by an alkylation reaction using an alkyl halide),
to yield a compound of formula (I/a) : 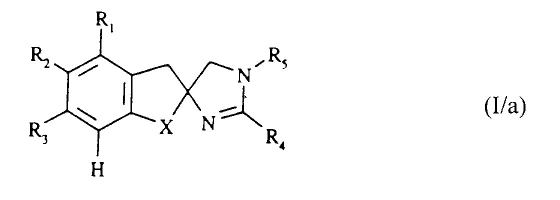 wherein X, R₁, R₂, R₃, R₄ and R₅ are as defined for formula (I), a particular case of compounds of formula (I),
which compound of formula (I/a)
is purified, if necessary, according to a conventional purification technique,
is separated, if desired, into its isomers according to a conventional purification technique, and is optionally converted into addition salts thereof with a pharmaceutically acceptable acid.

11. Process for the preparation of a compound of formula (I) according to claim 1 wherein Y represents an oxygen atom or a sulphur atom, characterised in that there is used as starting material a compound of formula (VI) according to claim 10,
which is reacted with formic acid in an anhydrous medium saturated with hydrochloric acid, to yield a compound of formula (VII) : 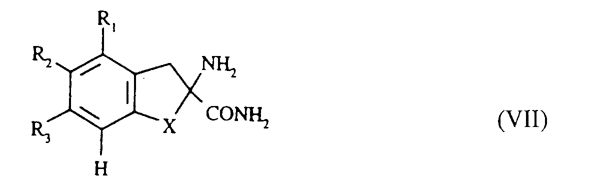 wherein R₁, R₂, R₃ and X are as defined for formula (I),
which is converted in concentrated hydrochloric acid medium into the corresponding acid of formula (VIII) : 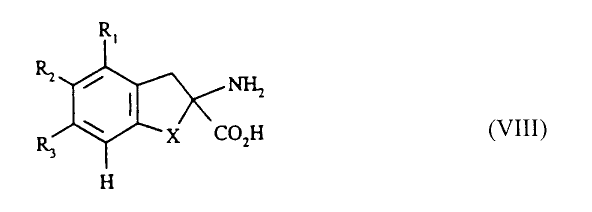 wherein R₁, R₂, R₃ and X are as defined for formula (I),
which is subjected to reduction with lithium aluminium hydride in inert medium to yield a compound of formula (IXa) : 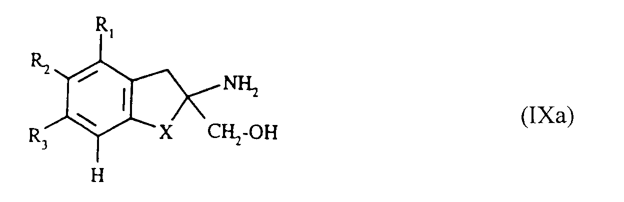 wherein R₁, R₂, R₃ and X are as defined for formula (I),
which compound of formula (IXa) is converted, depending on the nature of the desired compounds of formula (I), into the corresponding tosylate using para-toluenesulphonic acid and is then reacted with thiourea or thioacetic acid
to yield, after hydrolysis, a compound of formula (IXb) : 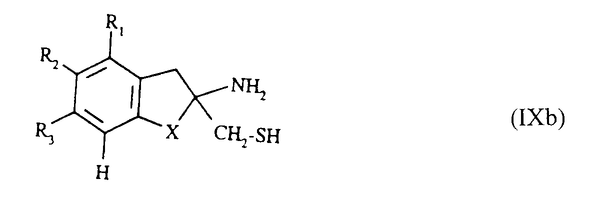 wherein R₁, R₂, R₃ and X are as defined for formula (I),
which compound of formula (IXa) or (IXb) is reacted with formamidine in alcoholic medium, an alkyl formate or with a cyanogen halide (followed, depending on the nature of the desired compound of formula (I), by an alkylation reaction using an alkyl halide),
to yield a compound of formula (I/b) 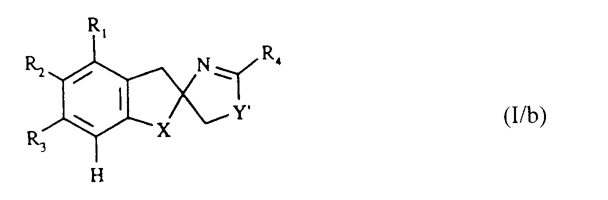 wherein R₁, R₂, R₃, R₄ and X are as defined above and Y' represents an oxygen atom or a sulphur atom, a particular case of compounds of formula (I),
which compound of formula (I/b)
is purified, if necessary, according to a conventional purification technique,
is separated, if desired, into its isomers according to a conventional purification technique, and is optionally converted into addition salts thereof with a pharmaceutically acceptable acid.

12. Pharmaceutical compositions comprising as active ingredient at least one compound according to any one of claims 1 to 9, alone or in combination with one or more pharmaceutically acceptable, inert, non-toxic excipients or carriers.

13. Pharmaceutical compositions according to claim 12 containing at least one active ingredient according to any one of claims 1 to 9 employed as an α₂-adrenergic agonist, as an anaesthetic, analgesic, hypotensive, sedative, vasoconstrictor or decongestant or to relieve the symptoms of opiate withdrawal.

## Patentansprüche

1. Verbindungen der Formel (I): 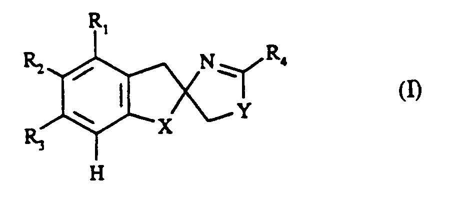 in der:
- X -CH₂-, -(CH₂)₂-, -CH=CH-, -O-CH₂-, -S-CH₂-, -SO-CH₂- oder -SO₂-CH₂- darstellt,
- Y ein Sauerstoffatom, ein Schwefelatom oder eine Gruppe -NR₅- darstellt,
- R₁ ein Halogenatom, eine geradkettige oder verzweigte (gegebenenfalls durch ein oder mehrere Halogenatome substituierte) (C₁-C₆)-Alkylgruppe, eine Hydroxylgruppe oder eine geradkettige oder verzweigte (C₁-C₆)-Alkoxygruppe darstellt,
- R₂ ein Wasserstoffatom, ein Halogenatom. eine geradkettige oder verzweigte (gegebenenfalls durch ein oder mehrere Halogenatome substituierte) (C₁-C₆)-Alkylgruppe, eine Hydroxylgruppe, eine geradkettige oder verzweigte (C₁-C₆)-Alkoxygruppe oder eine geradkettige oder verzweigte (C₁-C₆))-Alkylthiogruppe darstellt,
- R₃ ein Wasserstoffatom, ein Halogenatom, eine geradkettige oder verzweigte (gegebenenfalls durch ein oder mehrere Halogenatome substituierte) (C₁-C₆)-Alkylgruppe, eine Hydroxylgruppe, eine geradkettige oder verzweigte (C₁-C₆)-Alkoxygruppe oder eine geradkettige oder verzweigte (C₁-C₆)-Alkylthiogruppe darstellt,
- R₄ ein Wasserstoffatom oder eine (gegebenenfalls durch eine oder zwei geradkettige oder verzweigte (C₁-C₆)-Alkylgruppen substituierte) Aminogruppe darstellt,
- R₅ ein Wasserstoffatom oder eine geradkettige oder verzweigte (C₁-C₆)-Alkylgruppe darstellt,
oder
- R₁ und R₂ gemeinsam mit dem sie tragenden Kohlenstoffatom einen Benzolring bilden mit der Maßgabe, daß in diesem Fall X -CH₂- oder -(CH₂)₂- bedeutet,
deren Isomere sowie deren Additionssalze mit einer pharmazeutisch annehmbaren Säure.

2. Verbindungen der Formel (I) nach Anspruch 1, worin X-(CH₂)₂- darstellt, deren Isomere sowie deren Additionssalze mit einer pharmazeutisch annehmbaren Säure.

3. Verbindungen der Formel (I) nach Anspruch 1, worin Y -NH- darstellt, deren Isomere sowie deren Additionssalze mit einer pharmazeutisch annehmbaren Säure.

4. Verbindungen der Formel (I) nach Anspruch 1, worin Y ein Sauerstoffatom darstellt, deren Isomere sowie deren Additionssalze mit einer pharmazeutisch annehbmaren Säure.

5. Verbindungen der Formel (I) nach Anspruch 1, worin R₂ und R₃ gleichzeitig ein Wasserstoffatom darstellen, deren Isomere sowie deren Additionssalze mit einer pharmazeutisch annehmbaren Säure.

6. Verbindungen der Formel (I) nach Anspruch 1, worin R₄ ein Wasserstoffatom darstellt, deren Isomere sowie deren Additionssalze mit einer pharmazeutisch annehmbaren Säure.

7. Verbindung der Formel (I) nach Anspruch 1, in der R₄ eine Aminogruppe darstellt, deren Isomere sowie deren Additionssalze mit einer pharmazeutisch annehmbaren Säure.

8. Verbindung der Formel (I) nach Anspruch 1, nämlich Spiro[(1,3-diazacyclopent-1-en)-5:2'-(8'-chlor-1',2',3',4'-tetrahydronaphthalin)], dessen Isomere sowie dessen Additionssalze mit einer pharmazeutisch annehmbaren Säure.

9. Verbindung der Formel (I) nach Anspruch 1, nämlich Spiro[(1-oxa-2-amino-3-azacyclopent-2-en)-4:2'-(8'-chlor-1',2',3',4'-tetrahydronaphthalin)], dessen Isomere sowie dessen Additionssalze mit einer pharmazeutisch annehmaren Säure.

10. Verfahren zur Herstellung der Verbindungen der Formel (I) nach Anspruch 1, worin Y eine Gruppe -NR₅- darstellt, dadurch gekennzeichnet, daß man als Ausgangsprodukt ein Verbindung der Formel (II) verwendet: 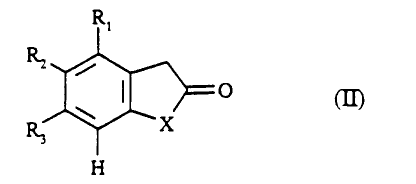 in der X, R₁, R₂ und R₃ die bezüglich der Formel (I) angegebenen Bedeutungen besitzen,
welche man:
- entweder mit Benzylamin in Gegenwart von p-Toluolsulfonsäure umsetzt zur Bildung der Verbindung der Formel (III): 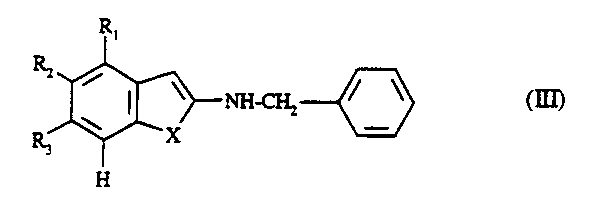 in der X, R₁, R₂ und R₃ die bezüglich der Formel (I) angegebenen Bedeutungen besitzen,
welche man unter einer inerten Atmosphäre und in Gegenwart von Zinkiodid mit Trimethylsilylcyanid umsetzt,
zur Bildung der Verbindung der Formel (IV): 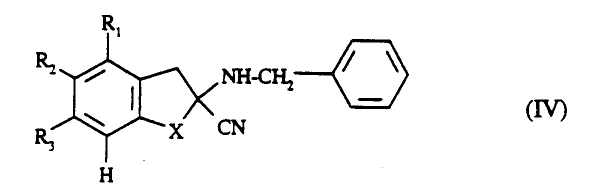 in der X, R₁, R₂ und R₃ die bezüglich der Formel (I) angegebenen Bedeutungen besitzen,
welche man mit Lithiumaluminiumhydrid und dann durch katalytische Hydrierung reduziert zur Bildung der Verbindung der Formel (V): 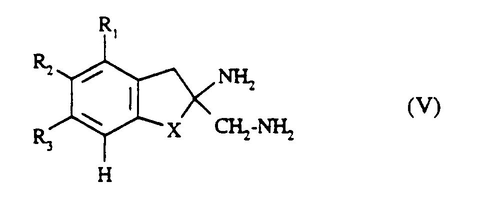 in der X, R₁, R₂ und R₃ die bezüglich der Formel (I) angegebenen Bedeutungen besitzen,
- oder mit Kaliumcyanid in Gegenwart von Ammoniumchlorid in inertem Medium oder mit Natriumcyanid in saurem Medium oder mit Trimethylsilylcyanid in Gegenwart von Zinkiodid und dann mit einer gesättigten alkoholischen Ammoniaklösung umsetzt
zur Bildung der Verbindung der Formel (VI): 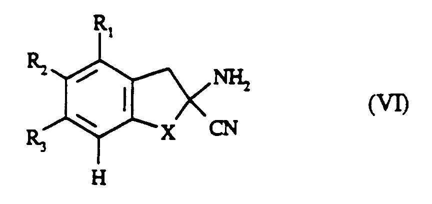 in der X, R₁, R₂ und R₃ die bezüglich der Formel (I) angegebenen Bedeutungen besitzen,
welche man mit Hilfe von Lithiumaluminiumhydrid reduziert zur Bildung der oben definierten Formel (V),
welche Verbindung der Formel (V) man
mit Formamidin in alkoholischem Medium, einem Alkylformiat oder mit einem Cyanogenhalogenid (gefolgt in Abhängigkeit von der Art der herzustellenden Verbindung der Formel (I) von einer Alkylierungsreaktion mit einem Alkylhalogenid) umsetzt
zur Bildung der Verbindung der Formel (I/a), einem Sonderfall der Verbindungen der Formel (I): 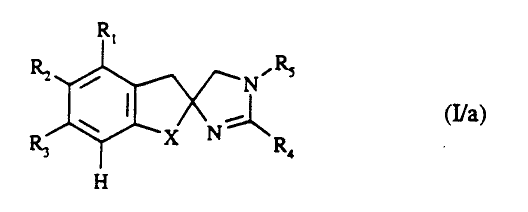 in der X, R₁, R₂, R₃, R₄ und R₅ die bezüglich der Formel (I) angegebenen Bedeutungen besitzen,
welche Verbindung der Formel (I/a) man
gegebenenfalls mit Hilfe einer klassischen Reinigungsmethode reinigt,
gewünschtenfalls gemäß einer klassischen Reinigungsmethode in ihre Isomeren auftrennt und
gegebenenfalls in ihre Additionssalze mit einer pharmazeutisch annehmbaren Säure überführt.

11. Verfahren zur Herstellung der Verbindungen der Formel (I) nach Anspruch 1, worin Y ein Sauerstoffatom oder ein Schwefelatom bedeutet, dadurch gekennzeichnet, daß man als Ausgangsprodukt eine Verbindung der Formel (VI) nach Anspruch 10 einsetzt,
welche man mit Ameisensäure in wasserfreiem mit Chlorwasserstoffsäure gesättigtem Medium umsetzt
zur Bildung der Verbindung der Formel (VII): 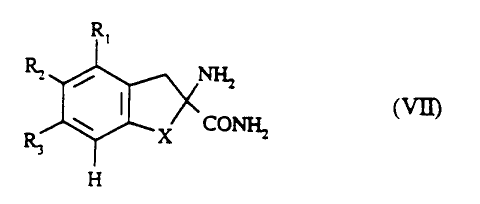 in der R₁, R₂, R₃ und X die bezüglich der Formel (I) angegebenen Bedeutungen besitzen,
welche man in konzentriertem Chlorwasserstoffmedium in die entsprechende Säure der Formel (VIII) umwandelt: 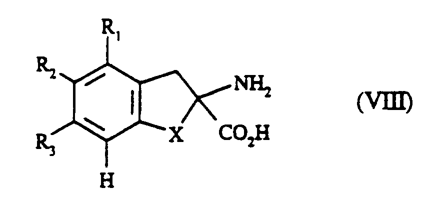 in der R₁, R₂, R₃ und X die bezüglich der Formel (I) angegebenen Bedeutungen besitzen,
welche man einer Reduktion mit Lithiumaluminiumhydrid in inertem Medium unterzieht zur Bildung der Verbindung der Formel (IXa): 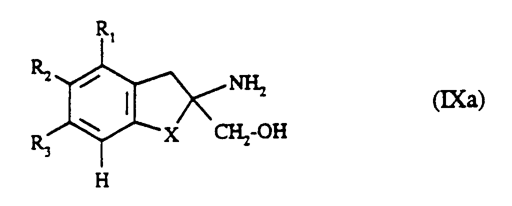 in der R₁, R₂, R₃ und X die bezüglich der Formel (I) angegebenen Bedeutungen besitzen,
welche Verbindung der Formel (IXa) man in Abhängigkeit von der Art der herzustellenden Verbindungen der Formel (I) mit Hilfe von p-Toluolsulfonsäure in das entsprechende Tosylat umwandelt und dann mit Thioharnstoff oder Thioessigsäure umsetzt,
so daß man nach der Hydrolyse die Verbindung der Formel (IXb) erhält: 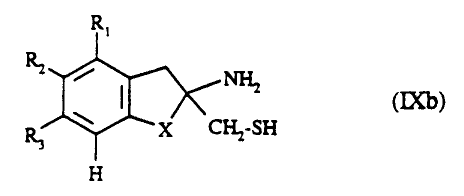 in der R₁, R₂, R₃ und X die bezüglich der Formel (I) angegebenen Bedeutungen besitzen,
welche Verbindung der Formel (IXa) oder (IXb) man mit Formamidin in alkoholischem Medium, einem Alkylformiat oder einem Cyanogenhalogenid (gefolgt in Abhängigkeit von der Art der herzustellenden Verbindung der Formel (I) einer Alkylierungsreaktion mit einem Alkylhalogenid) unterwirft,
zur Bildung der Verbindung der Formel (I/b), einem Sonderfall der Verbindungen der Formel (I): 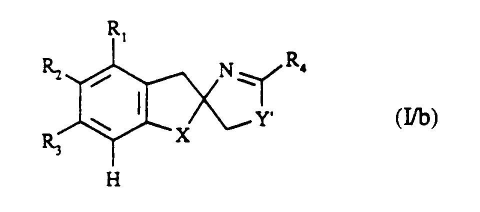 in der R₁, R₂, R₃, R₄ und X die oben angegebenen Bedeutungen besitzen und Y' ein Sauerstoffatom oder ein Schwefelatom darstellt,
welche Verbindung der Formel (I/b) man
gegebenenfalls mit Hilfe einer klassischen Reinigungsmethode reinigt,
gewünschtenfalls mit Hilfe einer klassischen Reinigungsmethode in die Isomeren auftrennt und
gegebenenfalls in ihre Additionssalze mit einer pharmazeutisch annehmbaren Säure überführt.

12. Pharmazeutische Zubereitungen, enthaltend als Wirkstoff mindestens eine Verbindung nach einem der Ansprüche 1 bis 9 allein oder in Kombination mit einem oder mehreren inerten, nichttoxischen, pharmazeutisch annehmbaren Trägermaterialien oder Bindemitteln.

13. Pharmazeutische Zubereitungen nach Anspruch 12, enthaltend mindestens einen Wirkstoff nach einem der Ansprüche 1 bis 9 zur Verwendung als α₂-adrenergischer Agonist, als Anästhetikum, Analgetikum, Hyopotensivum, Sedativum, gefäßverengendes Mittel, Abführmittel und zur Behandlung von Symptomen des Opiatentzugs.
